# EUROPEAN PATENT SPECIFICATION

(11) **EP 1 510 200 B1**
(45) Date of publication and mention of the grant of the patent: **11.10.2006**
(21) Application number: 03292096.9
(22) Date of filing: 26.08.2003
(51) Int. Cl.: A61Q 15/00

(54) **Stabilized antiperspirant compositions containing soy products**
Stabilisiertes Antitranspirantmittel Sojaprodukte enthaltend
Composition antiperspirante stabilisée comprenant de produits dérivés du soja

(43) Date of publication of application: 02.03.2005
(73) Proprietor: Johnson & Johnson Consumer France SAS, 92787 Issy les Moulineaux Cedex 9 (FR)
(72) Inventor: Magnant, Stephanie, Hameau de Verdun 27400 La Vacherie (FR); Jousselin, Magali, 27430 Saint-Pierre du Vauvray (FR); Quiry, Nathalie, 27400 Houndouville (FR)
(74) Representative: Weber-Bruls, Dorothée

(56) References cited:
- EP-A- 1 074 240
- WO-A-02/07697
- GB-A- 1 098 951
- US-A- 5 468 473
- DATABASE WPI Section Ch, Week 200118 Derwent Publications Ltd., London, GB; Class D21, AN 2001-172966 XP002266911 & JP 2000 351720 A (NOEVIR KK), 19 December 2000 (2000-12-19)
- DATABASE WPI Section Ch, Week 199102 Derwent Publications Ltd., London, GB; Class D16, AN 1991-012245 XP002266912 & JP 02 286165 A (KANEBO LTD), 26 November 1990 (1990-11-26)

## Description

### Brief description of the invention

This invention relates to antiperspirant compositions comprising soy products, one ore more antiperspirant agents such as aluminium chlorohydrate and a stabilizing system, which additionally influence hair growth.

### Background of the Invention

Antiperspirant products are typically applied to areas of the body where the concentration of sweat glands is higher, such as the armpits. These are areas which, to a lesser or greater extent, are covered with hair to provide more effective cooling. In recent times hair growth at these areas has become culturally less desirable and is considered as less attractive.

An antiperspirant product that additionally retards or stops hair growth would be attractive for consumers. It is an object of this invention to provide products that combine these two attributes.

Soy products and in particular non-denatured soy products are known to retard hair growth as is described in EP-A-1074240. The latter describes compositions and methods for delaying hair growth, reducing hair follicle and hair shaft size and hair shaft pigmentation by topically applying to the skin a composition comprising legume extracts including soymilk.

WO 01/34099 describes the use of non-denatured soy product containing compositions for depigmentation, evening out skin tone and skin texture, skin firming and care of the skin.

US-6,555,143 discloses compositions and methods that relate to legume products and in particular to soy products for regulating firmness of the skin, hair or nails; cleansing the skin, hair or nails; reducing and/or delaying hair or nail growth; and a number of other useful applications. EP-A-1236465 describes legume products having trypsin inhibitory activity, in particular soy products, having reduced microbial content and the use thereof in compositions for application on the skin, nails and hair.

WO02/07697 describes a mixture which contains hydrolysed soya protein and at least one extract of a plant which is selected from the group consisting of hypericum perforatum, hamamelis virginiana, arnica montana, and salix alba. This soy containing product is used in antiperspirants to inhibit hair growth.

It would have been attractive to combine an antiperspirant active such as aluminium chlorohydrate with soy products and in particular with non-denatured soy products. However, the latter are not stable in the presence of antiperspirant actives so that it was not possible to combine both types of actives in one product. Finding a stabilizing system that would allow this combination is an attractive goal to achieve and is the object of this invention.

Surprisingly it has been found that a stabilizing system comprising the association of analkoxylated fatty alcohol, a cellulose derivative and a silicate derivative results in formulations of good stability.

### Summary of the invention

The present invention is directed to an antiperspirant composition comprising a soy product and at least one topically acceptable antiperspirant active ingredient, wherein the composition further comprises :
a) an alkoxylated fatty alcohol;
b) a cellulose derivative;
c) a silicate derivative.

The soy product in particular is a non-denatured soy product, e.g. a non-denatured soymilk or powder. The antiperspirant in particular is selected from the group consisting of aluminium, zirconium salts, mixed aluminium/zirconium salts and complexes thereof.

In a particular aspect, the compositions of the invention contain alkoxylated alcohol incorporated in an amount from 0.1 to 10% maximum, cellulose derivatives in an amount from 0.01 to 3% and silicate derivatives in an amount from 0.01% to 3%.

In a further aspect the invention provides the use, and in particular the cosmetic use, of a composition as defined herein, reducing or stopping perspiration and deodorizing the skin.

Or, alternatively, the invention concerns a method, and in particular a cosmetic method, for reducing or stopping perspiration and deodorizing the skin, which method or cosmetic method comprises applying to the affected skin area an amount of a composition as defined herein.

### Detailed description of the invention

The compositions of the present invention contain soy products that may be in the form of a fluid (e.g., soymilk) or a solid (e.g., a soybean powder or soymilk powder). What is meant by "soy product" is a substance derived from the soybean, containing the ingredients naturally found in soybeans, at the relative concentrations as found in the beans. In preferred embodiments, the soy product is a non-denatured soy product. The latter is a soy product which has been obtained by processes that leave the active proteins intact by carefully controlling the process parameters such as the temperature, the extraction media. This can be measured, for example, by the presence of intact soybean trypsin inhibitor (STI) protein.

In another embodiment, the soy product is soymilk. One way to make soymilk is to soak the soybeans in deionized or purified water for several hours, and grind them after they were fully hydrated, with the addition of small quantities of water. (The grinding process allows the soybean milk to be extracted). After collection, the soybean milk may be filtered to remove any residual parts of the bean husk. The soymilk used in this invention can be fresh soymilk as described above, or may be made from soybean powder and water. The soybean powder is milled from soybeans and may also be lyophilized, spray dried, or freeze-dried and the resulting soymilk may or may not be filtered. Such prepared soymilk may have from about 1 to about 90% by weight dry soybean powder. Another example is the use of soymilk powder, made from lyophilized, spray dried or freeze-dried soymilk, with the addition of water and finished with or without filtration or homogenization.

Other methods of soybean extraction could also be used to create the active ingredients used in this invention. For example, but not limited to, the active ingredients could be extracted from ground soybeans using ethanol/water mixtures, followed by the removal of the ethanol from the extract, in such ways that the protease inhibitory activity of the soybean will be retained.

The compositions of the present invention may contain from about 1% to about 99%, by weight, of the soy product. For example, when a liquid soy product (e.g., soymilk) is used, the composition may contain from about 50% to about 99%, by weight, (e.g., from about 70% to about 99%) of the liquid soy product. For example, when a solid soy product (e.g., soybean powder or soymilk powder) is used, the composition may contain from about 1% to about 50%, by weight (e.g., from about 2% to about 30%, by weight) of the solid soy product. Compositions that comprise solid soy products may also comprise water (e.g., distilled water or water contained within soymilk) to form a liquid base to the composition (e.g., to form a cream, lotion or gel). Such composition may comprise from about 50% to about 98% by weight (e.g., from about 70% to about 98%, by weight) of water.

The soy products useful in this invention may be produced from all soybean species, regardless of their geographic origin, sun exposure, harvest time and the like. However, specific strains, geographic origins or growth conditions might be preferred.

For example, but not limiting to, soybean strains particularly rich in their trypsin inhibitor (e.g. STI, LTI, BBI) content or growth conditions that result in trypsin inhibitor enrichment in the bean, ought be preferred. It should be noted that the soy products useful in the compositions of this invention have a distinctive odor, which may be tolerable in some cultures, but is undesired in others. If necessary, the odor of the compositions of this invention may be reduced by using soybean products derived from specific strains of soybeans known to produce reduced odor, including, but not limited to, lipoxygenase-2-deficient beans and those having modified sugar profile, and the like. A process to reduce oxygen levels in the formulation may also reduce the odor. Various masking agents or fragrances may also be used to mask the odor.

Preferred for use in the compositions of the present invention are non-denatured soy products. These are preferably decontaminated as described in EP-1236465, for example by gamma irradiation of non-denatured soymilk powder, preferably at a dose of about 10 kGy.

The compositions of the invention further contain antiperspirant agents, in particular antiperspirant metal salts, also referred to as astringent metal salts, especially the inorganic and organic salts of aluminium, zirconium and zinc, as well as mixtures thereof. These comprise salts such as aluminium halides, aluminium halohydrates, aluminium hydroxyhalides, zirconyl oxyhalides, zirconyl hydroxyhalides, and mixtures thereof. Preferred halides are the chlorides. Of particular interest are the groups of aluminium hydroxy chlorodydrates and the zirconyl hydroxy chlorohydrates.

Two groups of preferred aluminium salts for use in the antiperspirant emulsions include those that can be represented by the formula: Al₂(OH)ₐCl₆₋ₐ.*x*H₂O wherein a is from 2 to 5; x is from 1 to 6; and wherein a and x may have non-integer values. Of particular interest are the aluminium chlorohydroxides wherein a=5 or a=4.
Preferred zirconium salts for use in the antiperspirant emulsions include those that can be represented by the formula ZrO(OH)₂₋ₐClₐ.*x*H₂O wherein a is any number having a value of from 0 to 2; x is from about 1 to about 7; and wherein a and x may have non-integer values. Preferably the zirconium salts are used as combined aluminium/zirconium halides or as complex halohydrate salts, which may be with amino acids such as glycine. Of particular interest are complexes that contain aluminium chlorhydroxide and zirconyl hydroxy chloride, in particular combined with glycine, conforming to the above described formulas.

Preferred antiperspirant actives comprise aluminium chlorohydrates, aluminium-zirconium-chlorohydrate as well as zinc salts. Other such agents comprise aluminium hydroxylactates as well as acid aluminium/zirkonium salts. A particularly suitable chlorohydrate is the compound of formula [Al₂(OH)₅Cl]·2.5 H₂O, also referred to as aluminium chlorhydrol or aluminium hydroxychloride. Further such agents are aluminium-zirconium-tetrachlorohydroxy-glycine-complexes. Specific species include aluminium chlorohydrate, aluminium dichlorohydrate, aluminium sesquichlorohydrate, aluminium chlorohydrex propylene glycol complex, aluminium dichlorohydrex propylene glycol complex, aluminium sesquichlorohydrex propylene glycol complex, aluminium chlorohydrex polyethylene glycol complex, aluminium dichlorohydrex polyethylene glycol complex, aluminium sesquichlorohydrex polyethylene glycol complex, aluminium zirconium trichlorohydrate, aluminium zirconium tetrachlorohydrate, aluminium zirconium pentachlorohydrate, aluminium zirconium octachlorohydrate, aluminium zirconium trichlorohydrex glycine complex, aluminium zirconium tetrachlorohydrex glycine complex, aluminium zirconium pentachlorohydrex glycine complex, aluminium zirconium octachlorohydrex glycine complex, aluminium chloride, aluminium sulfate buffered, and combinations thereof.

The antiperspirant actives are commercially available or can be prepared by methods known in the art.

The compositions according to the present invention can also comprise 0.01 to 90% of a deodorant active. The deodorant active used in the cosmetics of the invention can be any deodorant active known in the art such as alcohols, in particular aliphatic monohydric alcohols such as ethanol or propanol, antimicrobial actives such as polyhexamethylene biguanides or chlorinated aromatics, eg triclosan, non-microbiocidal deodorant actives such as triethylcitrate, bactericides and bacteriostatis. Yet other deodorant actives can include zinc salts such as zinc ricinoleate.

Esterase inhibitors can be added as further deodorizing agents, i.e. agents such as trialkyl citrates such as trimethylcitrates, tripropyl citrates, triisopropyl citrates, tributyl citrates and in particular triethyl citrates. Further esterase inhibitors are sterol sulfates or -phosphates, such as, for example, lanosterine-, cholesterine-, campesterine-, stigmasterine- and sitosterine sulfate respectively -phosphate, dicarbonic acids and their esters, such as, for example, glutaric acid, glutaric acid monoethylester, glutaric acid diethylester, adipinic acid, adipinic acid monoethylester, adipinic acid diethylester, malonic acid and malonic acid diethylester, hydroxycarbonic acids and their esters such as, for example, citric acid, malonic acid, tartaric acid or tartaric acid diethylester.

Antibacterial active ingredients that influence the growing conditions and eradicate perspiration decomposing bacteria, or impede their growth, can also be present in the lipid and/or aqueous phase. Examples of such ingredients are triclosan, phenoxyethanol and chlorohexidine gluconate and in particular 5-chloro-2-(2,4-dichlorophenoxy)-phenol.

The compositions according to the invention additionally contain a stabilizing system, which is a combination of an emulsifying agent, more specifically an alkoxylated fatty alcohol, and gelling agents, more specifically a cellulose derivative and a silicate derivative. This system allows the stabilization of the association of an antiperspirant agent such as aluminium chlorohydrate and a soy product, in particular a non-denatured soy product.

It is believed that the specific association of ethoxylated alcohol, cellulose derivatives and silicate derivatives creates a specific network that stabilizes the soy product and the antiperspirant agent and maintains both the soy product and the antiperspirant agent in a stable formulation.

The alkoxylated fatty alcohols are derived from fatty alcohols, which comprise in particular C₁₂-C₂₄-fatty alcohols, more in particular the C₁₆-C₂₂-fatty alcohols, still more in particular the C₁₆-C₂₀-fatty alcohols, preferably in particular the C₁₆-C₁₈-fatty alcohols, that are derived from natural fats, oils or waxes such as, for example, myristyl alcohol, 1-pentadecanol, cetylalcohol, 1-heptadecanol, stearyl alcohol, 1-nonadecanol, arachidyl alcohol, 1-heneicosanol, behenyl alcohol, brassidyl alcohol, lignoceryl alcohol, ceryl alcohol, myricyl alcohol, lauryl alcohol, capryl alcohol, caprinyl alcohol, cetyl alcohol, palmoleyl alcohol, isostearyl alcohol, oleyl alcohol, elaidyl alcohol, petroselinyl alcohol, arachidyl alcohol, gadoleyl alcohol, erucyl alcohol, including mixtures thereof such as cetearyl alcohol, C_{12/13} fatty alcohol, as well as Guerbet alcohols. Preferred for use in the present invention, are saturated, straight or branch chained fatty alcohols. However also unsaturated, straight or branch chained alcohols can be used, optionally in a mixture with saturated alcohols.

Mixtures of fatty alcohols can evidently also be used, including fatty alcohol fractions obtained from the reduction of the corresponding fatty acid fractions derived from naturally occurring oils or fats such as, for example, almond oil, soybean oil, sunflower oil, safflower oil, corn oil, canola oil, borage oil, evening primrose oil, grapeseed oil, wheat germ oil, avocado oil, jojoba oil, sesame oil, walnut oil, linseed oil, palm oil, olive oil, castor oil, macadamia oil, rapeseed oil, peanut oil, coconut oil, and turnip seed oil. The fatty alcohols from which these products are derived can be saturated, straight or branch chained fatty alcohols. However also unsaturated, straight or branch chained alcohols can be used, optionally in a mixture with saturated alcohols.

The alkoxylated fatty alcohols may contain from 1 to 50 alkoxy units, preferably from 1 to 30 alkoxy units, more preferably from 10-25 alkoxy units. The alkoxy units preferably are ethoxy or propoxy units. Particularly preferred alkoxylated fatty alcohols are ethoxylated or propoxylated C₁₆₋₁₈ fatty alcohols. Of particular interest are the C₁₆₋₁₈ fatty alcohols such as cetyl alcohol, stearyl alcohol, isostearyl alcohol and mixtures thereof such as cetearyl alcohol, more in particular the ethoxylated analogs having the degree of alkoxylation outlined above. It may be advantageous to use ethoxylated fatty alcohols with different degrees of ethoxylation, for example a mixture of ethoxylated fatty alcohols with low degree of ethoxylation, e.g. with 2-6 ethoxy units, and of ethoxylated fatty alcohols relatively high degree of ethoxylation, e.g. with 15-25 ethoxy units.

Suitable cellulose derivatives comprise hydroxy alkyl celluloses, in particular hydroxyethyl and hydroxypropylcellulose.

Suitable silicates for the compositions of the present invention comprise gelling/thickening silicates and include magnesium aluminium silicate, bentonite, hectorite and derivatives thereof. Magnesium aluminium silicate occurs naturally in such smectite materials as colerainite, saponite and sapphire. Refined magnesium aluminium silicate useful herein is commercially available under the trade name Veegum^{™}. Modified magnesium aluminium silicate materials such as magnesium aluminium silicate mineral CMC are available under the trade name Veegum Plus^{™}. This modified clay material contains smectitie clay with sodium carboxy methyl cellulose and titanium dioxide. Bentonite is a native hydrated colloidal aluminium silicate clay. Hectorite is one of the montmorillonite minerals that is a principal consituent of bentonite clay.

In certain embodiments, the stabilizing system is a combination of an ethoxylated fatty alcohol, a hydroxy ethyl cellulose and magnesium silicate.

The compositions of this invention may contain further stabilizing components. The latter may for example comprise one or more components selected from the group consisting of one or more antioxidants, chelating agents and preservatives.

In preferred embodiments, the stabilizing system comprises :
a) from 2-6% of an alkoxylated fatty alcohol, preferably of an ethoxylated fatty alcohol;
b) from 0.2-0.8% of a cellulose derivative, preferably a hydroxyalkylcellulose;
c) from 0.5-1% of a silicate derivative, preferably a gellable silicate derivative.

In further preferred embodiments, the stabilizing system comprises :
a) from 2-6% of an ethoxylated fatty alcohol;
b) from 0.2-0.8% of a hydroxyethylcellulose;
c) from 0.5-1% of magnesium aluminium silicate.

Particular embodiments of this invention comprise compositions containing :
a) a non-denatured soy product;
b) an aluminium chlorohydrate anti-perspirant;
c) an ethoxylated C₁₆-C₂₀-fatty alcohol;
d) hydroxyethylcellulose;
e) magnesium aluminium silicate.

Further particular embodiments of this invention comprise compositions comprising:
a) from 0.1% to 5% of a non-denatured soy product;
b) from 10 to 40% of an aluminium chlorohydrate anti-perspirant;
c) from 2-6% of an ethoxylated C₁₆-C₂₀-fatty alcohol;
d) from 0.2-0.8% of hydroxyethylcellulose;
e) from 0.5-1% magnesium aluminium silicate.

Particularly interesting compositions in accordance with the present invention are those which are based on an emulsion which can be an oil-in-water, water-in-oil or a complex emulsion. Particular embodiments are compositions comprising an oil-in-water emulsion which contain a soy product, an antiperspirant active agent and a stabilizing system which contains:
a) an alkoxylated fatty alcohol;
b) a cellulose derivative;
c) a silicate derivative; wherein the quantities of the components can be as outlined in this specification.

The compositions of this invention may contain one or more preservatives. Preservatives are useful for substantially preventing microbial decomposition. Examples of preservatives include phenoxyethanol and parabens such as methylparaben, ethylparaben, and propylparaben. Other examples of preservatives are listed on pages 1654- 55 of the International Cosmetic Ingredient Dictionary and Handbook, eds. Wenninger and McEwen (CTFA, 7th ed., 1997), hereinafter referred to as the "Cosmetic Handbook." The compositions may comprise from about 0.01% to about 20%, by weight (more preferably, from about 0.5% to about 5%, by weight) of preservative. Microbial contamination can also be eliminated by gamma irradiation or microfiltration, or by brief heat treatments that do not result in the elimination of protease inhibitory activity.

Antioxidants and/or chelating agents may also be used to increase shelf life and stability of the compositions. Antioxidants may be added both for formulation stabilization and for biological efficacy. Antioxidant compounds and their derivatives include, but are not limited to, water-soluble antioxidants such as sulfhydryl compounds and their derivatives (e.g., sodium metabisulfite and N-acetyl-cystein), lipoic acid and dihydrolipoic acid, resveratrol, acetyl-cysteine (IneferineTM) or lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g. retinol and retinyl palmitate), tocopherols (e.g., tocopherol acetate), tocotrienols, and ubiquinone. Natural extracts containing antioxidants suitable for use in the compositions of this invention, include, but are not limited to, extracts containing flavonoids and isoflavonoids and their derivatives (e.g., genistein and diadzein), extracts containing resveratrol and the like. Examples of such natural extracts include grape seed, green tea, pine bark, propolis, and legume extracts. Other examples of antioxidants may be found on pages 1612-13 of the Cosmetic Handbook. The compositions of the present invention may comprise the antioxidant in an amount of from about 0.001% to about 20%, by weight (e.g., from about 0.01% to about 10% by weight) of the composition.

Chelating agents may also be useful in assisting the stabilization of the compositions of this invention. Examples of chelating agents include EDTA and derivatives thereof (e.g., disodium EDTA and dipotassium EDTA), Iniferine TM, lactoferrin, and citric acid. Other examples of chelating agents are listed on page 1626 of the Cosmetic Handbook.

The compositions of the present invention may comprise the chelating agent in an amount of from about 0.001% to about 20%, by weight (e.g., from about 0. 01% to about 10% by weight) of the composition.

Thickening agents (e.g., thickeners or viscosity enhancing agents) may be utilized in the compositions of this invention to alter their viscosity. The desired viscosity of the composition will depend upon the intended use (e.g., as a bath product, cream, lotion, or gel). For example, in applications such as bath or wash products, the viscosity of the composition should be relatively low, similar to an aqueous solution. Application as a cream, lotion, or gel will have slightly higher viscosity (e.g., between about 100 mPa·s and 100,000 mPa·s.

Thickening agents that can be added to the compositions of this invention to alter viscosity include polymers such as polyacrylates (e.g., polyacrylamide). Other examples of viscosity modifying agents are listed on pages 1692-97 of the Cosmetic Handbook. To achieve the appropriate viscosity, compositions of the present invention may comprise from about 0.01 % to about 20%, by weight (e.g., from about 0.1 % to about 5%, by weight) of a thickening agent.

The compositions of the invention are prepared by adding the aqueous formulations of the salts to the soy component or vice versa. The compositions of the present invention therefore are mostly of aqueous nature.

In the compositions of the present invention the w/w ratio of the antiperspirant active to the soy product may vary but in particular is in the range of about 50 : 1 to 1 : 1, further in particular from 30 : 1 to 2 : 1, still further in particular from 20 : 1 to 5 : 1, preferably from about 15 : 1 to 5 : 1, more preferably the w/w ratio is in the range of about 10 : 1 to 5 : 1. These w/w ratios relate to the total amount of dry soy product and dry salts.

The composition may contain further ingredients or additives such as active ingredients, surfactants, emulsifiers, consistency factors, conditioners, emollients, skin caring ingredients, moisturizers, lubricants, fillers, binding agents, anti-oxidants, preservatives, fragrances and the like.

The soy product preferably is used in the compositions of the invention at concentrations from 0.001% to 10% and preferably from 0.1% to 5%, more preferably from 0.5 % to 3 %. The antiperspirant actives taken together should be used in the compositions between about 1% to 60% and preferably from about 5 to 40%, more preferably from about 10 % to 40 %.

Unless indicated otherwise, all percentages in the preceding and following paragraphs are w/w percentages.

The formulations according to the present invention can be prepared by mixing the appropriate ingredients. It is also possible to make premixes and to add ingredients or other premixes. In a preferred method of preparation, the compositions of this invention are emulsion-based, in particular oil-in-water emulsions and are made by preparing an aqueous phase containing all hydrophilic components, an oil phase containing all lipophilic components, and subsequently adding the oil phase to the aqueous phase as to prepare an emulsion. Preferably, a premix of the soy product in some water is added after the formation of the emulsion.

The compositions of the invention are effective to effect changes in hair growth, hair follicle and hair shaft size and hair shaft pigmentation.

Soy products and in particular non-denatured soy products as mentioned herein, are known to have a number of beneficial effects such as increasing skin firmness and elasticity, even tone and texture, to combat and treat the effects of skin aging, to prevent and treat sun-induced damage and acne. The compositions of the invention may therefore have these additional benefits.

The compositions according to the invention may take a variety of forms, which will depend upon the nature of the end product used. The compositions may be in the form of a solution, a hydrophilic lotion, an ointment, a cream or a gel. The formulations may also be, for example, in the form of oil-in-water, water-in-oil or multiple emulsions or foaming products.

The antiperspirant compositions of the invention can be applied using a range of different applicators depending on the individual preferences of consumers, including aerosols, roll-ons, pump sprays, sticks and so-called mushroom applicators which are used to apply cream formulations, wax-based sticks, soap-based sticks, compressed powder sticks, roll-on suspensions or solutions, emulsions, gels, creams, squeeze sprays, pump sprays, and aerosols. Each product form contains its own selection of additional components, some essential and some optional. The types of components typical for each of the above product forms may be incorporated in the corresponding compositions of the invention. Preferred are sticks, gels and creams or soft solids. The term stick traditionally indicates a bar of material with a solid appearance which is usually housed within a dispensing container and which retains its structural integrity and shape while being applied. When a portion of the stick is drawn across the skin surface a film of the stick composition is transferred to the skin surface. Although the stick has the appearance of a solid article capable of retaining its own shape for a period of time, the material usually has a structured liquid phase so that a film of the composition is readily transferred from the stick to another surface upon contact.

For use, the stick is applied directly to the skin of the user. This contrasts with soft solid compositions that exist as a stick within their container, but whereby for use a portion is extruded from the container through apertures which have smaller cross section than the body of composition which is being made to flow out through these apertures.

Compositions for sticks will be structured so that the compositions retain their shape. Compositions for roll-on applications will be liquid or semi-liquid, whereas sprayable compositions have lower viscosity.

The following example is meant to illustrate the invention.

### Examples

In the following formulation example all percentages are by weight (w/w).

The formulation is an oil-in-water formulation which was prepared in the following manner. First an aqueous phase was made by adding hydroxyethyl cellulose, magnesium aluminium silicate to the required amount of water. Subsequently disoudium EDTA, methylparaben and propylparaben are added to this mixture. Separately an oily phase was made by adding steareth-21 and steareth-2, glyceryl laurate to the dicapryl carbonate. Then the oily phase was added to the aqueous phase while stirring at increased temperature (75 - 80 °C). The whole is allowed to stir for a while to allow forming a stable emulsion. Then the aluninium chlorohydrate is added to the thus obtained emulsion and a premix consisting of non-denatured soy powder which was decontaminated by gamma irradiation, PEG-8, phenoxyethanol and citronellyl methylcrotonate is added to the emulsion, under stirring.

| | % Active | % (w/w) | % (w/w) Active |
|---|---|---|---|
| Water | | 38.8875 | |
| Hydroxyethylcellulose | | 0.5500 | |
| Magnesium aluminium silicate | | 0.8000 | |
| Aluminium Chlorohydrate 50%/ aqua 50% ('Chlorhydrol 50%') | 50 | 40.0000 | 20 |
| Dicapryl carbonate | | 5.0000 | |
| Steareth-21 | | 3.0000 | |
| Steareth-2 | | 1.0000 | |
| Glyceryl laurate | | 0.6000 | |
| Disodium EDTA | | 0.1000 | |
| Methyl Paraben | | 0.3000 | |
| Propyl Paraben | | 0.1000 | |
| Phenoxyethanol | | 0.8000 | |
| Glycine soja | | 2.5125 | |
| Methyl dihydrojasmonate | | 1.0000 | |
| Citronellyl Methylcrotonate | | 0.3500 | |
| PEG 8 | | 5.0000 | |
| Total | | 100.000 | |

## Claims

1. An antiperspirant composition comprising a soy product and at least one topically acceptable antiperspirant active ingredient, wherein the composition further comprises :
a) an alkoxylated fatty alcohol;
b) a cellulose derivative;
c) a silicate derivative.

2. A composition according to claim 1, wherein the soy product is a non-denatured soy product, e.g. a non-denatured soymilk or powder.

3. A composition according to claim 1, wherein the antiperspirant is selected from aluminium and zirconium salts.

4. A composition according to claim 3, wherein the antiperspirant is an aluminium chlorohydrate.

5. A composition according to claim 1, wherein the alkoxylated fatty alcohol is an ethoxylated C₁₆₋₂₀ fatty alcohol.

6. A composition according to claim 1, wherein the cellulose derivative is hydroxyethylcellulose.

7. A composition according to claim 1, wherein the silicate derivative is magnesium aluminum silicate.

8. A composition according to claims 1-7, wherein the composition contains alkoxylated alcohol incorporated in an amount from 0.1 to 10% maximum, cellulose derivatives in an amount from 0.01 to 3% and silicate derivatives in an amount from 0.01% to 3%, all percentages being w/w relative to the total weight of the composition.

9. An antiperspirant composition according to claim 1, wherein the composition comprises :
a) from 2-6% of an ethoxylated fatty alcohol;
b) from 0.2-0.8% of a hydroxyethylcellulose;
c) from 0.5-1% of magnesium aluminium silicate.

10. An antiperspirant composition according to claim 1, wherein the composition comprises :
a) from 0.1% to 5% of a non-denatured soy product;
b) from 10 to 40%, an aluminium chlorohydrate anti-perspirant;
c) from 2-6% of an ethoxylated C₁₆-C₂₀-fatty alcohol;
d) from 0.2-0.8% of hydroxyethylcellulose;
e) from 0.5-1% magnesium aluminium silicate.

## Patentansprüche

1. Antitranspirantzusammensetzung, welche ein Sojaprodukt und zumindest einen äußerlich akzeptablen Antitranspirantwirkstoff umfaßt, **dadurch gekennzeichnet, daß** die Zusammensetzung weiterhin umfaßt:
a) einen alkoxylierten Fettalkohol;
b) ein Zellulosederivat;
c) ein Silikatderivat.

2. Zusammensetzung nach Anspruch 1, **dadurch gekennzeichnet, daß** das Sojaprodukt ein nicht-denaturiertes Sojaprodukt ist, zum Beispiel eine nicht-denaturierte Sojamilch oder ein nicht-denaturiertes Sojapulver.

3. Zusammensetzung nach Anspruch 1, **dadurch gekennzeichnet, daß** das Antitranspirant aus Aluminium- und Zirkoniumsalzen ausgewählt wird.

4. Zusammensetzung nach Anspruch 3, **dadurch gekennzeichnet, daß** das Antitranspirant ein Aluminiumchlorhydrat ist.

5. Zusammensetzung nach Anspruch 1, **dadurch gekennzeichnet, daß** der alkoxylierte Fettalkohol ein ethoxylierter C₁₆₋₂₀ Fettalkohol ist.

6. Zusammensetzung nach Anspruch 1, **dadurch gekennzeichnet, daß** das Zellulosederivat Hydroxyethylzellulose ist.

7. Zusammensetzung nach Anspruch 1, **dadurch gekennzeichnet, daß** das Silikatderivat Magnesiumaluminiumsilikat ist.

8. Zusammensetzung nach den Ansprüchen 1 - 7, **dadurch gekennzeichnet, daß** die Zusammensetzung alkoxylierten Alkohol in einer Menge von 0,1 bis maximal 10 %, Zellulosederivate in einer Menge von 0,01 bis 3 % und Silikatderivate in einer Menge von 0,01 bis 3 % enthält, wobei alle Prozentsätze w/w relativ zum Gesamtgewicht der Zusammensetzung sind.

9. Antitranspirantzusammensetzung nach Anspruch 1, **dadurch gekennzeichnet, daß** die Zusammensetzung
a) von 2 bis 6 % eines ethoxylierten Fettalkohols;
b) von 0,2 bis 0,8 % einer Hydroxyethylzellulose;
c) von 0,5 bis 1 % Magnesiumaluminiumsilikat umfaßt.

10. Antitranspirantzusammensetzung nach Anspruch 1, **dadurch gekennzeichnet, daß** die Zusammensetzung
a) von 0,1 bis 5 % eines nicht-denaturierten Sojaprodukts;
b) von 10 bis 40 % eines Aluminiumchlorhydrat-Antitranspirants;
c) von 2 bis 6 % eines ethoxylierten C₁₆₋₂₀ Fettalkohols;
d) von 0,2 bis 0,8 % Hydroxyethylzellulose;
e) von 0,5 bis 1 % Magnesiumaluminiumsilikat umfaßt.

## Revendications

1. Composition anti-perspirante comprenant un produit de soja et au moins un ingrédient actif anti-perspirant localement acceptable, dans laquelle la composition comprend en plus :
a) un alcool gras alcoxylé ;
b) un dérivé de cellulose ;
c) un dérivé de silicate.

2. Composition selon la revendication 1, dans laquelle le produit de soja est un produit de soja non dénaturé, par exemple, une poudre ou un lait de soja non dénaturé.

3. Composition selon la revendication 1, dans laquelle l'agent anti-perspirant est choisi à partir de sels d'aluminium et de zirconium.

4. Composition selon la revendication 3, dans laquelle l'agent anti-perspirant est un chlorhydrate d'aluminium.

5. Composition selon la revendication 1, dans laquelle l'alcool gras alcoxylé est un alcool gras en C₁₆₋₂₀ éthoxylé.

6. Composition selon la revendication 1, dans laquelle le dérivé de cellulose est une hydroxyéthylcellulose.

7. Composition selon la revendication 1, dans laquelle le dérivé de silicate est un silicate d'aluminium et de magnésium.

8. Composition selon les revendications 1 à 7, dans laquelle la composition comprend un alcool alcoxylé incorporé en une quantité de 0,1 à 10 % au maximum, des dérivés de cellulose en une quantité de 0,01 à 3 % et des dérivés de silicate en une quantité 0,01 à 3 %, tous les pourcentages étant en poids par rapport au poids total de la composition

9. Composition anti-perspirante selon la revendication 1, dans laquelle la composition comprend :
a) de 2 à 6 % d'un alcool gras éthoxylé ;
b) de 0,2 à 0,8 % d'une hydroxyéthylcellulose ;
c) de 0,5 à 1 % d'un silicate de magnésium et d'aluminium.

10. Composition anti-perspirante selon la revendication 1, dans laquelle la composition comprend :
a) de 0,1 % à 5 % d'un produit de soja non dénaturé ;
b) de 10 à 40 % d'un agent anti-perspirant au chlorhydrate d'aluminium ;
c) de 2 à 6 % d'un alcool gras en C₁₆-C₂₀ éthoxylé ;
d) de 0,2 à 0,8 % d'une hydroxyéthylcellulose ;
e) de 0,5 à 1 % d'un silicate de magnésium et d'aluminium.
